(19) **Europäisches Patentamt**
**European Patent Office**
**Office européen des brevets**

(11) **EP 0 974 272 B1**

(12) **EUROPEAN PATENT SPECIFICATION**

(45) Date of publication and mention
of the grant of the patent:
**01.08.2007 Bulletin 2007/31**

(51) Int Cl.:
*A23L 1/236* (2006.01)    *A23L 1/22* (2006.01)
*C07K 5/06* (2006.01)

(21) Application number: **97942214.4**

(22) Date of filing: **01.10.1997**

(86) International application number:
**PCT/JP1997/003500**

(87) International publication number:
**WO 1998/039979 (17.09.1998 Gazette 1998/37)**

(54) **SWEETENER COMPOSITION IMPROVED IN TASTE**

SÜSSSTOFFZUSAMMENSETZUNG MIT VERBESSERTEM GESCHMACK

COMPOSITION D'ÉDULCORANT AU GOÛT AMÉLIORÉ

(84) Designated Contracting States:
**AT BE CH DE DK ES FI FR GB GR IE IT LI NL PT SE**

(30) Priority: **10.03.1997 JP 5458297**
**10.03.1997 JP 5458397**

(43) Date of publication of application:
**26.01.2000 Bulletin 2000/04**

(73) Proprietor: **Ajinomoto Co., Inc.**
**Tokyo 104 (JP)**

(72) Inventors:
• **ISHII, Shoichi,**
**Food R&D Lab. Ajinomoto Co. Inc.**
**Kanagawa-ken 210 (JP)**
• **SHIMIZU, Tetsuji,**
**Food R&D Lab. Ajinomoto Co. Inc.**
**Kanagawa-ken 210 (JP)**

(74) Representative: **Nash, David Allan**
**HASELTINE LAKE,**
**Redcliff Quay**
**120 Redcliff Street**
**Bristol BS1 6HU (GB)**

(56) References cited:
**WO-A-94/11391          JP-A- 8 038 099**
**JP-A- 8 503 026          JP-A- 9 051 777**
**US-A- 4 158 068**

• **AYYA N ET AL: "QUANTITATIVE AND QUALITATIVE EVALUATION OF HIGH-INTENSITY SWEETENERS AND SWEETENER MIXTURES" , CHEMICAL SENSES,IRL PRESS, OXFORD,GB, VOL. 17, NR. 3, PAGE(S) 245-259 XP000991203 ISSN: 0379-864X * page 245, paragraph 1 * * page 251, paragraph 1 * * page 254, paragraph 1 ***

**Description**

Technical Field

The present invention relates to a sweetner composition having a natural sweetness and therefore being excellent in its quality, which comprises N-[N-(3,3-dimethylbutyl)-L-$\alpha$-aspartyl]-L-phenylalanine 1-methyl ester (hereinafter, abbreviated as "DMB-APM") which is a sweetener having a high level of sweetness, together with Aspartame (hereinafter, abbreviated as "APM") which is an amino acid-based sweetener, and optionally Acesulfame K (hereinafter, abbreviated as "AceK").

Background Art

It is reported that the level of sweetness of DMB-APM which is a sweetener having a high level of sweetness is 10,000 times that of sweetness of sucrose (Publication of the Japanese translation of an international patent application No. 503,206/1996 and international patent application No. PCT /FR93/01103) in terms of weight ratio, and that the level of sweetness of APM which is an amino acid-based sweetener is approximately 200 times that of sweetness of sucrose (Japanese Patent Publication kokoku No. 31,031/1972) in terms of weight ratio (these weight ratios vary depending on their concentration, the kinds of coexistent ingredients, and the like).

The profile of the sweetness characteristics of DMB-APM have not been reported in detail. According to the present inventors' findings, however, the sweetener is extremely weak in early taste (which means that a sweetener when put in the mouth tastes sweet as early as sucrose), whereas it is extremely strong in later taste (which means that a sweetener tastes sweet later than sucrose). Further, it has a strong astrigent taste. Accordingly, the sweetness characteristics of DMB-APM are not so well-balanced as those of sucrose. Meanwhile, APM is weak in early taste though not so weak as DMB-APM, whereas it is strong in later taste though not so strong as DMB-APM. Accordingly, in case sucrose is defined as having a natural sweetness, both of DMB-APM and APM have such strange sweetness characteristics that their early taste is weak and their later taste is strong.

With respect to the improvements in the sweetness characteristics of APM, various proposals have been made mainly on the improvements in the later taste (for example, Japanese Patent Application Laid-Open (Kokai) Nos. 148,255/1981, 141,760/1983 and 220,668/1983). Further, there is a proposal on a method of obtaining a natural sweetness closer to the sweetness of sucrose with the combined use of APM with sucrose (Japanese Patent Application Laid-Open (Kokai) No. 152,862/1982).

On the other hand, AceK (tne sweetness level being, like APM, approximately 200 times that of sucrose in terms of weight ratio) is inferior in sweetness quality to DMB-APM and APM. It is stronger in later taste, bitter taste, astringent taste, peculiar taste and irritation, compared with DMB-APM and APM. A variety of improvements including an improvement in sweetness with the combined use of AceK with APM (U.S. Patent No. 4,158,068) have been proposed.

In the above-described background of the prior art, it is an object of the present invention to provide, in view of the above-mentioned sweetness characteristics of DMB-APM, DMB-APM in the form of a sweetner composition which has a high level of sweetness and whose sweetness quality is closer to that of sucrose, by improving the sweetness characteristics of DMB-APM, which can, in turn, be realized by strengthening the early taste, and weakening the later taste as well as the astringent taste.

US-A-4 158 068 teaches a sweetener mixture having an improved saccharose-like taste consisting of acetosulfame and at least a further artificial sweetener, which is selected especially from the aspartyl peptide esters.

WO-A-9 411 391 teaches the use of derivatives of aspartame such as N-[N-(3,3-dimethylbutyl)-L-$\alpha$- aspartyl]-L-phenylalanine 1-methyl ester as an intense sweetener composition for foods and beverages, alone or combined with other sweeteners, such as aspartame and acesulfame.

Disclosure of Invention

The present inventors have assiduously conducted investigations to achieve the above-mentioned object, and have consequently found that a sweetner composition whose sweetness characteristics are such well-balanced that the early taste is strengthened (i.e., to the degree that the composition when put in the mouth tastes sweet as early as sucrose), the later taste is weakened (i.e., to the degree that it tastes sweet, when put in the mouth, as early as sucrose), and the astringent taste is weakened, can be obtained by combining DMB-APM which is a sweetener having a high level of sweetness with APM which is an amino acid-based sweetener and optionally AceK at specific ratios. These findings have led to the completion of the present invention.

It is easily considered that the early taste of a sweetener having a weak early taste can be improved upon using concurrently a sweetener having a strong early taste (for example, glycyrrhizin). In practice, however, things are not so easy. According to the prior knowledge, it is very difficult to anticipate that the early taste of DMB-APM as well as that of APM can be both improved by combining DMB-APM with APM having an early taste which is weak though not so weak as that of DMB-APM, that consequently the early taste given by the combination of DMB-APM and APM can be better than that of DMB-APM or APM when used singly, and such combination, in turn, can give a sweetner composition having wholly well-balanced sweetness characteristics and a sweetness closer to that of sucrose, and that a sweetner composition having well-balanced sweetness characteristics can be obtained by combining DMB-APM, and optionally APM, with AceK which is inferior in sweetness characteristics.

The sweetner composition of the present invention contains DMB-APM and APM and optionally AceK at a ratio where the sweetness quality closer to that of sucrose than that of each sweetener when singly used, can be obtained.

In case a sweetner composition of the present invention comprises as the sweetening ingredients DMB-APM and APM, the amount of DMB-APM is between 0.1% and 35% by weight based on the total amount of the DMB-APM and the APM. With respect to the composition in which the amount of DMB-APM is less than 0.1% or more than 35%, no improvements in the early taste, the later taste and the astringent taste can be provided even by the combined use, compared with those these sweeteners give when used singly. Accordingly, when the amount of DMB-APM based on the total amount of DMB-APM and APM is between 0.1% and 35% by weight, sweetner compositions having improved sweetness characteristics can be obtained. More preferably, when the amount is between 0.3% and 3% by weight, sweetner compositions which have very well-balanced sweetness characteristics and whose sweetness quality is closer to that of sucrose can be provided.

Likewise, the combined use of DMB-APM and APM with AceK can give a sweetner composition having improved sweetness characteristics. In this case, the amount of AceK based on the total amount of DMB-APM, APM and AceK is between 0.5% and 85% by weight, preferably between 1% and 75% by weight, whereby the early taste can be strengthened, the later taste can be weakened and the astringent taste can be weakened. Thus, a sweetner composition having well-balanced sweetness characteristics and plenty of body can be obtained.

In case a sweetner composition comprises as the sweetening ingredients DMB-APM and AceK in combination, the amount of DMB-APM is between 3% and 80% by weight based on the total amount of DMB-APM and AceK. When the amount of DMB-APM is less than 3% or more than 80%, no improvements in the early taste, the later taste and the astringent taste can be provided even by the combined use, compared with those these sweeteners give when used singly. Accordingly, when the amount of DMB-APM based on the total amount of DMB-APM and AceK is between 3% and 80% by weight, sweetner compositions having the improved sweetness characteristics can be obtained. More preferably, when it is between 3.5% and 60% by weight, sweetner compositions which have well-balanced sweetness characteristics and whose sweetness quality is closer to that of sucrose can be provided.

The sweetner composition of the present invention can of course be in distribution in the form of a powdery mixture comprising DMB-APM together with APM and optionally AceK. For the purpose of making use easy, like conventional sweetner compositions having a high level of sweetness, it can contain a diluent or bulking agent such as sugar alcohol, oligosaccharide, dietary fiber and the like, or can be prepared into granules, tablets or the like. Such diluent and bulking agent include sweeteners having a low level of sweetness such as sucrose, glucose and the like.

The sweetner composition of the present invention can, like a conventional sweetener having a high level of sweetness such as APM or the like, be incorporated into various foods and drinks such as table sweeteners, carbonated and non-carbonated beverages, frozen desserts, jellies, cake, bread, candies, chewing gum, an oral composition including tooth paste, medications and the like, and it can provide natural and well-balanced sweetness characteristics closer to those of sucrose, as compared with conventional sweetner compositions having a high level of sweetness. Above all, the composition of the present invention is quite useful in various beverages including carbonated beverages with respect to which DMB-APM is tasted as solution when they are drunk.

Best Mode for Carrying Out the Invention

Hereinbelow, the present invention will be more concretely explained, with refernce to the following test examples and examples.

Test Example 1

The tests were conducted using solutions whose solvent was pure water obtained by ion-exchanging and distilling tap water. The said ion-exchanged and distilled water will be referred to simply as "pure water".

An aqueous solution of DMB-APM having a sweetness level equivalent to that of aqueous 10% sucrose solution (such sweetness level being, hereinafter, referred to as "10% PSE (Point of subjective equality)") was prepared, and compared with aqueous 10% sucrose solution with respect to 9 items, namely, early taste, peculiar taste, heavy taste, irritation, bitter taste, later taste, astringent taste, clear taste and round taste. As a result, it was found that the DMB-APM solution was extremely weak in early taste, extremely strong in later taste, and strong in astringent taste. Further, the peculiar taste and the heavy taste were strong. Thus, the balance of the sweetness characteristics was greatly lost.

Likewise, aqueous 10% PSE APM solution (i.e., aqueous APM solution with APM dissolved therein in an amount to give 10% PSE) was compared with aqueous 10% sucrose solution. The former exhibited such sweetness characteristics that the early taste was weak, though not so weak as that of the DMB-APM solution, and the later taste was strong, though not so strong as that of the DMB-APM solution.

Meanwhile, several aqueous solutions of DMB-APM and APM combined were prepared in such way that the ratios of DMB-APM to APM were distributed within the range of from 1:9 to 9:1 in terms of sweetness ratio, with 10% PSE being kept constantly. They were then compared with aqueous 10% sucrose solution. Consequently, it was found that they were all strengthened in early taste, weakened in later taste, and reduced in astringent taste. Although the peculiar taste and the bitter taste were somewhat given to the solutions, their sweetness quality was greatly improved, while their sweetness characteristics were well-balanced.

Test Example 2

A carbonated cola solution with DMB-APM dissolved therein in an amount to give 10% PSE was prepared, and compared with another carbonated cola solution with sucrose dissolved therein in a 10% concentration. The former solution was weakened in peculiar taste and heavy taste, but was weak in early taste and strong in later taste and astringent taste, as compared with the latter solution, when the comparative results of the aqueous non-carbonated solutions given in Test Example 1 were taken into consideration.

Likewise, a carbonated cola solution with APM dissolved therein in an amount to give 10% PSE was prepared, and compared with another carbonated cola solution with sucrose dissolved therein in a 10% concentration. The former solution exhibited such sweetness characteristics that the early taste was weak, the later taste was strong and the round taste was weak, though not so weak, not so strong and not so weak, respectively, as in the case of the carbonated cola solution having DMB-APM dissolved therein.

Meanwhile, several carbonated cola solutions of DMB-APM and APM combined were prepared in such way that the ratios of DMB-APM to APM were distributed within the range of from 1:9 to 9:1 in terms of sweetness ratio, with 10% PSE being kept constantly. They were then compared with another carbonated cola solution with sucrose dissolved therein in a 10% concentration. Consequently, it was found that the former solutions were all strengthened in early taste, and weakened in later taste and astringent taste. Thus, they were greatly improved in sweetness quality, while their sweetness characteristics were well-balanced. Especially when the DMB-APM to APM ratios were between 5:5 and 1:9, the early taste was strengthened to the same degree as that of sucrose, and the well-balanced sweetness characteristics were given.

Test Example 3

As in Test Example 2, a carbonated cola solution with DMB-APM dissolved therein in an amount to give 5% PSE was prepared and compared with another carbonated cola solution with sucrose dissolved therein in a 5% concentration. The former solution was weaker in early taste, and stronger in later taste and astringent taste, as compared with the latter solution.

Meanwhile, several carbonated cola solutions of DMB-APM and APM combined were prepared in such way that the ratios of DMB-APM to APM were distributed within the range of from 1:9 to 9:1 in terms of sweetness ratio, with 5% PSE being kept constantly. They were then compared with another carbonated cola solution with sucrose dissolved therein in a 5% concentration. Consequently, it was found that the former solutions were all strengthened in early taste, and weakened in later taste and astringent taste. Thus, they were greatly improved in sweetness quality, while their sweetness characteristics were well-balanced. Especially when the ratio of APM was higher within the range, as in Test Example 2, the early taste was strengthened to the same degree as that of sucrose, and the well-balanced sweetness characteristics were provided.

Test Example 4

Concurrent use of a mixture of DMB-APM and APM with AceK was studied.

Several carbonated cola solutions of DMB-APM, APM and AceK combined were prepared in such way that the ratios of the total amount of DMB-APM and APM to the amount of AceK were distributed within the range of from 1:9 to 9:1 in terms of sweetness ratio, with 10% PSE being kept constantly. They were then compared with another carbonated cola solution with sucrose dissolved therein in a 10% concentration. In this case, the sweetness ratio of DMB-APM to APM was also changed. When the ratio of the total amount of DMB-APM and APM to the amount of AceK was between 9:1 and 6:4 (i.e., when the ratio of AceK was 40% or less in terms of PSE), the solutions in which the three sweeteners dissolved were strengthened in early taste and weakened in astringent taste, providing the improved sweetness quality. Especially a higher ratio of APM to DMB-APM in terms of sweetness ratio gave marked improvements in sweetness quality, while well-balanced sweetness characteristics were provided (for example, when the DMB-APM: APM:AceK ratio was 2:7:1 in terms of PSE).

When the ratio of AceK was high, the early taste was somewhat strengthened, but the bitter taste, the peculiar taste, the heavy taste, the irritation and the later taste were extremely strengthened. Further, the clear taste and the round taste were extremely weakened. Thus, the well-balanced sweetness characteristics were lost.

Example 1

An aqueous solution of DMB-APM was prepared from a more concentrated DMB-APM solution by diluting to a 10% PSE concentration (i.e., a concentration equivalent to 10% PSE), assuming that the sweetness level of DMB-APM was 8,000 times that of sucrose. Separately, aqueous sucrose solutions having sucrose concentrations of (a) 6.94%, (b) 8.33%, (c) 10.0%, (d) 12.0% and (e) 14.4% were prepared. The aqueous DMB-APM solution and the sucrose solutions were given to a panel of 20 panelist (hereinafter, referred to as "n = 20") in order to organoleptically evaluate the sweetness level of the first mentioned solution by determining to which sucrose solution it was the closest in sweetness level.

When Solutions (a), (b), (c), (d) and (e) were chosen, points 1, 2, 3, 4 and 5 were respectively given thereto in this order, and the average score given from the scores of the 20 panelists was 2.4. The sweetness level of the aqueous DMB-APM solution was 9.0% PSE according to the following equation (1).

$$(10.0 - 8.33) \times (2.4 - 2) + 8.33 = 9.0 \qquad \cdots (1)$$

Accordingly, the sweetness level of DMB-APM was 7,200 (= 8,000 × 9/10) times that of sucrose.

On the basis of this result, an aqueous 10% PSE DMB-APM solution (i.e., an aqueous DMB-APM solution having a sweetness level of 10% PSE, or an aqueous DMB-APM solution with DMB-APM dissolved therein in an amount to give 10% PSE) was prepared (by taking up 13.9 mg of DMB-APM, followed by filling up to 1,000 ml with pure water). And, an aqueous 10% sucrose solution was prepared (by taking up 100 g of sucrose, followed by filling up to 1,000 ml with pure water).

The aqueous 10% PSE DMB-APM solution was compared with the aqueous 10% sucrose solution as the control, with respect to 9 items, namely, early taste, peculiar taste, heavy taste, irritation, bitter taste, later taste, astringent taste, clear taste and round taste (n = 20) . As a result, it was found that the aqueous DMB-APM solution was extremely weak in early taste, extremely strong in later taste and strong in astringent taste (n = 20).

An aqueous 10% PSE solution of DMB-APM and APM combined (in a 5:5 ratio in terms of PSE) was prepared. In greater details, 6.9 mg (=5/7,200 (g/dl)) of DMB-APM and 395 mg of APM were taken up and filled up to 100 ml with pure water, followed by diluting ten (10)-fold with pure water. This aqueous solution had a sweetness level of 10% PSE (n = 20). In this solution, the amount of the DMB-APM based on the total amount of the DMB-APM and APM was 1.7%. With respect to the relationship between the APM weight(g) and the sweetness level (%PSE), the following equation(2) was used (this being same hereinafter).

$$Y \ (\%PSE \ sweetness \ level) = 46.06 \times (APM \ weight(g))^{0.687} \qquad \cdots (2)$$

The aqueous 10% PSE solution of DMB-APM and APM mixed (in a 5:5 ratio in terms of PSE) was compared with an aqueous 10% sucrose solution as a control, with respect to 9 items, namely, early taste, peculiar taste, heavy taste, irritation, bitter taste, later taste, astringent taste, clear taste and round taste.

As a result, it was found that the sweetness quality was improved by a combination of DMB-APM and APM. I.e., the early taste was strengthened, the later taste was weakened, and the astringent taste was weakened, which, in turn, provided well-balanced sweetness characteristics(n = 20).

Example 2

It is very difficult to organoleptically evaluate the sweetness level of a carbonated solution correctly. Accordingly, with respect to a carbonated cola solution of the present invention, a solution before carbonated (hereinafter, referred to as "a cola base solution") was adjusted in sweetness level (to 10% PSE), followed by carbonating. With respect to carbonated cola solutions thus prepared, comparison of the sweetness quality was carried out.

(1) Preparation of a cola base solution :

Into a 1,000-ml messflask are put 0.25 g of citric acid (crystal), 0.1 g of sodium citrate, 0.3 g of 85% phosphoric acid, 2 ml of a cola base perfume, and 1 ml of cola essence and a desired, calculated amount of the sweetener(s) is added thereto. The mixture is filled up to 1,000 ml with pure water (pH 2.8).

(1b) Preparation of sucrose cola base solutions:

5(five) sucrose cola base solutions were prepared by repeating Preparation (1) above, except that (a) 69.4 g, (b) 83.3 g, (c) 100 g, (d) 120 g and (e) 144 g of sucrose were respectively used as the sweetner.

(2) Preparation of a 10% PSE DMB-APM cola base solution:

13.9 mg of DMB-APM was put into a 100-ml messflask, and filled up with pure water (presumed PSE being 100%). A DMB-APM cola base solution was prepared by repeating Preparation (1) above, expect that the DMB-APM solution was employed as the sweetener (presumed PSE being 10%).

The DMB-APM cola base solution was organoleptically evaluated, as in Example 1, by determing to which sucrose cola base solution it was the closet in sweetness level. The average score was found to be 2.45 (n = 20), and the calculated PSE value was 9.1%. Therefore, the sweetness level of DMB-APM was 6,600 times that of sucrose in the cola base solution.

Then, another 10% PSE DMB-APM solution was prepared. In greater details, 15.2 mg of DMB-APM was put into a 100-ml messflask, and filled up with pure water (presumed PSE being 100%). The desired DMB-APM cola base solution was prepared by repeating Preparation (1) above, except that the DMB-APM solution was employed as the sweetener.

The last-mentioned DMB-APM cola base solution was organoleptically evaluated, as in Example 1, by determing to which sucrose cola base solution it was the closet in sweetness level. The average score was found to be 2.75 (n = 20), and a sweetness level of 10% PSE was identified.

(2a) Preparation of a 10% PSE cola base solution of DMB-APM and APM combined (at a 5:5 ratio in terms of PSE):

7 mg of DMB-APM and 395 mg of APM were put into a 100-ml messflask, and filled up with pure water (presumed PSE geing 100%). A cola base solution was prepared by repeating Preparation (1) above, except that the aqueous solution of DMB-APM and APM combined was employed as the sweetener. The sweetness level of this cola base solution was measured as in Example 1. The average score was 2.58, and a sweetness level of 9.3% PSE was identified.

Then, another 10% PSE DBM-APM plus APM solution was prepared. In greater details, 7.5 mg of DMB-APM and 425 mg of APM were charged into a 100-ml messflask, and filled up with pure water (presumed PSE being 100%). The desired cola base solution was prepared by repeating Preparation (1) above, except that the DMB-APM puls APM solution was employed as the sweetener. The sweetness level of the last-mentioned cola base solution was organoleptically evaluated as in Example 1. The average score was found to be 3.17 (calculated PSE value being 10%). In this cola base solution, the amount of the DMB-APM based on the total amount of the DMB-APM and APM was 1.7%.

(3) Preparation of a carbonated cola solution:

1,000 ml of each of the above-obtained cola base solutions was charged into a carbonation bomb, and carbon dioxide gas was charged therein. The bomb was allowed to stand overnight in a refrigerator, whereby the cola base solution was enough carbonated. When it was cooled well, the bomb was opened while it stood still, and the solution thereof was immediately charged into a 240-ml can.

(4) Evaluation of sweetness quality:

The carbonated, 10% PSE DMB-APM cola solution obtained under (3) above was compared with a carbonated, 10% sucrose cola solution as a control, with respect to 9 items, namely, early taste, peculiar taste, heavy taste, irritation, bitter taste, later taste, astringent taste, clear taste and round taste. As a result, it was found that the carbonated DMB-APM cola solution was of such sweetness quality that the early taste was extremely weak, the later taste was extremely strong, and the astringent taste was strong (n = 20).

The catbonated 10% PSE cola solution of DMB-APM and APM combined (at a 5:5 ratio in terms of PSE), which had been obtained under (3) above, was compared with a carbonated, 10% sucrose cola solution as the control, with respect to 9 items, namely, early taste, peculiar taste, heavy taste, irritation, bitter taste, later taste, astringent taste, clear taste and round taste. As a result, it was found that the carbonated DMB-APM plus APM cola solution (the DMB-APM and the APM being in a 5:5 ratio in terms of PSE) was of such improved sweetness quality that the early taste was strengthened, the later taste was weakened, and the astringent taste was weakened. Thus, well-balanced sweetness characteristics were provided (n = 20), being different from the carbonated DMB-APM cola solution.

Example 3

(1) Preparation of an aqueous 10% PSE solution of DMB-APM, APM and AceK combined (at a 4:4:2 ratio in terms of PSE):

5.6 mg( = 4/7,200 (g/dl)) of DMB-APM, 285 mg of APM and 49 mg of AceK were taken up, and filled up to 1,000 ml with pure water. This aqueous solution was organoleptically evaluated as in Example 1, with respect to the sweetness characteristics. The average score was 3.1 (n = 20), and a sweetness level of 10% PSE was identified.

In this solution, the amount of the DMB-APM based on the total amount of the DMB-APM, APM and AceK was 1.8%. With respect to the relationship of the AceK weight(g) and the sweetness level (%PSE), the following equation (3) was used (this being the same hereinafter).

$$\text{Y (\% PSE sweetness level)} = 19.09 \times (\text{AceK weight(g)})^{0.424}$$

$$\cdots (3)$$

The aqueous 10% PSE solution of DMB-APM, APM and AceK (at a 4:4:2 ratio in terms of PSE) was compared with an aqueous 10% sucrose solution as the control, with respect to 9 items, namely, early taste, peculiar taste, heavy taste, irritation, bitter taste, later taste, astringent taste, clear taste and round taste. As a result, the sweetness quality was improved although the bitter taste somewhat remained. The early taste was strengthened, the later taste was weakened, and the astringent taste was weakened, which, in turn, provided well-balanced sweetness characteristics (n = 20) .

(2) Preparation of a 10% PSE cola base solution of DMB-APM, APM and AceK combined (at a 4:4:2 ratio in terms of PSE):

A cola base solution (presumed PSE being 10%) was prepared by repeating the preparation of a cola base solution as given under Example 2 (1) above, except that 6.1 mg( = 4/6,600 (g/dl)) of DMB-APM, 285 mg of APM and 49 mg of AceK were taken up, and the whole mass was employed as the sweetener.
This cola base solution was organoleptically evaluated as in Example 1. The average score was 3.67 (n = 20), and the sweetness level was 11.3% PSE.
Then, a 10% PSE cola base solution was prepared again. In greater details, the cola base solution (presumed PSE being 10%) was prepared by repeating the preparation of a cola base solution as given under Example 2 (1) above, except that 5.4 mg of DMB-APM, 252 mg of APM and 43 mg of AceK were taken up, and the whole mass was employed as the sweeteners.
The last-mentioned cola base solution was organoleptically evaluated as in Example 1. The average score was 3.2, and the sweetness level was 10% PSE as calculated. In this solution, the amount of the DMB-APM based on the total amount of the DMB-APM, APM and AceK was 1.8%.

(3) Preparation of a carbonated cola solution:

1,000 ml of the cola base solution obtained under (2) above was charged into a carbonation bomb, and carbon dioxide gas was charged therein. The bomb was allowed to stand overnight in a refrigerator, whereby the cola base solution was enough carbonated. When it was cooled well, the bomb was opened while it stood still, and the solution thereof was immediately charged into a 240-ml can.

(4) Evaluation of sweetness quality:

The carbonated 10% PSE cola solution of DMB-APM, APM and AceK combined (at a 4:4:2 ratio in terms of PSE), which had been obtained under (3) above, was compared with a carbonated, 10% sucrose cola solution as the control, with respect to 9 items, namely, early taste, peculiar taste, heavy taste, irritation, bitter taste, later taste, astringent taste, clear taste and round taste. As a result, the sweetness quality was improved, i.e., the early taste was strengthened, the later taste was weakened, and the astringent taste was weakened, which, in turn, provided well-balanced sweetness characteristics (n = 20).

Example 4

Cola drinks were prepared by repeating the procedure as given under Example 3 (3) above, which contained the sweeteners in the ratios given in the following Table 1.

Table 1

| Run No. | DMB-APM:APM | | DMB-APM amount based on the total amount |
|---|---|---|---|
| | PSE ratio (%) | weight ratio (mg/dl) | (%) |
| 1 | 1:9 | 0.15:92.9 | 0.16 |
| 2 | 2:8 | 0.24:62.3 | 0.38 |
| 3* | 5:5 | 0.75:42.5 | 1.7 |
| 4 | 8:2 | 1.2:10.4 | 10.3 |
| 5 | 9:1 | 1.4:3.8 | 27.0 |
| 6** | 4.5:0.5 | 0.68:1.38 | 33.0 |

\* Described in Example 2.
\** In case of 5% PSE.

Each of the carbonated cola solutions of Run Nos. 1, 2 and 4 to 6 was compared with a carbonated, 10% sucrose cola solution as a control, with respect to 9 items, namely, early taste, peculiar taste, heavy taste, irritation, bitter taste, later taste, astringent taste, clear taste and round taste. As a result, being different from a corbonated cola solution in which DMB-APM was only used as the sweetener, they were all of such improved sweetness quality that the early taste was strengthend, the later taste was weakened, and the astringent taste was weakened, which, in turn, provided well-balanced sweetness characteristics. By the way, higher APM ratios gave higher improvements in sweetness quality (n = 20).

Example 5

Carbonated cola solutions were prepared by repeating the procedure as given under Example 3 (3) above, which contained the sweeteners in the ratios given in the following Table 2.

Table 2

| Run No. | DMB-APM:APM:AceK | | DMB-APM amount based on the total amount |
|---|---|---|---|
| | PSE ratio (%) | weight ratio (mg/dl) | (%) |
| 1 | 7:2:1 | 1.1:10.4:1.0 | 8 |
| 2 | 2:7:1 | 0.3:56.0:0.8 | 1.4 |
| 3 | 1:8:1 | 0.15:78.2:0.95 | 1.2 |
| 4* | 4:4:2 | 0.5:25.1:4.3 | 14.4 |
| 5 | 4:2:4 | 0.6:10.4:25.1 | 69.5 |
| 6 | 2:4:4 | 0.3:28.5:25.1 | 46.6 |
| 7 | 4:1:5 | 0.6:3.8:42.4 | 90.6 |
| 8 | 1:1:8 | 0.15:3.8:169.2 | 97.7 |

\* Described in Example 3.

Each of the carbonated cola solutions of Run Nos. 1 to 3 and 4 to 8 was compared with a carbonated, 10% sucrose cola solution as a control, with respect to 9 items, namely, early taste, peculiar taste, heavy taste, irritation, bitter taste, later taste, astringent taste, clear taste and round taste. As a result, with respect to all the solutions of Run Nos. 1 to 3, 5 and 6 (AceK ratio in terms of PSE being between 1 and 4), they were all of such improved sweetness quality that the early taste was strengthened, the later taste was weakened, and the astringent taste was weakened, which, in turn, provided well-balanced sweetness characteristics, being different from a carbonated cola solution in which DMB-APM was only used as the sweetener. By the way, higher APM ratios gave higher improvements in sweetness quality.

With respect to the solutions of Run Nos. 7 and 8 (AceK ratio in terms of PSE being 5 or more), although they were all strengthened in early taste, they were extremely strong in later taste, astringent taste, bitter taste and peculiar taste, and were extremely weakened in clear taste and round taste, which, in turn, provided imbalanced sweetness characteristics. Especially when the AceK ratio became increased, the balance of the sweetness characteristics was greatly lost (n = 20).

Example 6 (Comparative example)

A aqueous 10% PSE solution of DMB-APM and AceK (at a 8:2 ratio in terms of PSE) was compared with an aqueous 10% sucrose solution as the control, with respect to 9 items, namely, early taste, peculiar taste, heavy taste, irritation, bitter taste, later taste, astringent taste, clear taste and round taste. As a result, it was found that DMB-APM was improved in sweetness quality, when used together with AceK, i.e., the early taste was strengthened, the later taste was weakened, and the astringent taste was weakened, which, in turn, provided well-balanced sweetness characteristics (n = 20).

Example 7 (Comparative example)

(1) Preparation of a 10% PSE cola base solution of DMB-APM and AceK combined (at a 8:2 ratio in terms of PSE):

A cola base solution (presumed PSE being 10%) was prepared by repeating the preparation of a cola base solution as given under Example 2 (1) above, except that 11 mg of DMB-APM and 49 mg of AceK were taken up as the sweetener into a 1,000-ml messflask. The cola base solution was organoleptically evaluated as in Example 2. The average score was 2.05, and the sweetness level was 8.4% PSE. Therefore, another cola base solution was prepared in which the DMB-APM and AceK were contained in increased amounts by calculation to give a sweetness level of 10% PSE. In greater detail, for the last-mentioned cola base solution were employed as the sweetener $13( = 11 \times 10/8.4)$ mg of DMB-APM and $58( = 49 \times 10/8.4)$ mg of AceK. The sweetness level of this cola base solution was identified as 10% PSE, as described above. In this solution, the amount of the DMB-APM based on the total amount of the DMB-APM and AceK was 18.3%.

(2) Preparation of a carbonated cola solution:

1,000 ml of the cola base solution obtained above was charged into a carbonation bomb, and carbon dioxide gas was charged therein. The bomb was allowed to stand overnight in a refrigerator, whereby the cola base solution was enough carbonated. When it was cooled well, the bomb was opened while it stood still, and the solution thereof was immediately charged into a 240-ml can.

(3) Evaluation of sweetness quality:

The carbonated 10% PSE cola solution of DMB-APM and AceK combined (at a 8:2 ratio in terms of PSE), which had been obtained under (2) above, was compared with a carbonated, 10% sucrose cola solution as the control, with respect to 9 items, namely, early taste, peculiar taste, heavy taste, irritation, bitter taste, later taste, astringent taste, clear taste and round taste. As a result, it was found that DMB-APM when used together with AceK was improved in sweetness characteristics. I.e., the early taste was strengthened, the later taste was weakened, and the astringent taste was weakened, which, in turn, provided well-balanced sweetness characteristics (n = 20).

Example 8 (Comparative exemple)

Carbonated cola solutions were prepared by repeating the procedure as in Example 7 above, which contained the sweeteners in the ratios given in the following Table 3.

Table 3

| Run No. | DMB-APM:AceK | | DMB-APM amount based on the total amount |
|---|---|---|---|
| | PSE ratio (%) | weight ratio (mg/dl) | (%) |
| 1 | 9:1 | 1.4:1.0 | 58.3 |
| 2* | 8:2 | 1.3:5.8 | 18.3 |
| 3 | 6:4 | 1.1:30.5 | 3.5 |
| 4 | 5:5 | 0.8:42.4 | 1.9 |
| 5 | 2:8 | 0.4:169.2 | 0.2 |
| 6 | 1:9 | 0.15:169.7 | 0.1 |
| 7** | 4.5:0.5 | 0.68:0.19 | 78.2 |

\* Described in Example 7.
\*\* In case of 5% PSE.

Each of the carbonated cola solutions of Run Nos. 1, 3 and 7 was compared with a carbonated, 10% sucrose cola solution as a control, with respect to 9 items, namely, early taste, peculiar taste, heavy taste, irritation, bitter taste, later taste, astringent taste, clear taste and round taste. As a result, it was found that DMB-APM when used together with AceK was improved in sweetness characteristics. I.e., the early taste was strengthened, the later taste was weakened, and the astringent taste was weakened, -which, in turn, provided well-balanced sweetness characteristics were provided (n = 20).

Further, each of the carbonated cola solutions of Run Nos. 4 to 6 (AceK ratio in terms of PSE being 5 or more) was likewise compared with a carbonated, 10% sucrose cola solution as the control. As a result, it was found that DMB-APM when used together with AceK was somewhat improved in sweetness characteristics. In greater details, with respect to all the solutions, although the early taste was strengthened, the later taste, the astringent taste, the bitter taste and the peculiar taste were extremely strong, and the clear taste and the round taste were extremely weak. Thus, imbalanced sweetness characteristics were provided. Especially when the sweetness level ratio of AceK became increased, the balance of the sweetness characteristics was greatly, lost (n = 20).

Industrial Applicability

According to the present invention, a sweetner composition having a high level of sweetness and well-balanced sweetness characteristics is provided which cannot be provided upon using DMB-APM, APM or AceK singly. Diluents or bulking agents (including sucrose and the like) ordinarily used in this field may also be incorporated in the sweetner composition of the present invention. Its superiority is exhibited especially in carbonated cola solutions. The sweetner composition of the present invention is, however, applicable to all usages as a composition having improved sweetness quality.

**Claims**

1.  A sweetener composition containing N-[N-(3,3-dimethylbutyl)-L-$\alpha$-aspartyl]-L-phenylalanine 1-methyl ester (DMB-APM) and Aspartame (APM) and optionally containing Acesulfame K (AceK), in a ratio which gives sweetness quality thereto closer to that of sucrose than any one of N-[N-(3,3-dimethylbutyl)-L-$\alpha$-aspartyl]-L-phenylalanine 1-methyl ester, Aspartame and Acesulfame K.

2.  A sweetener composition according to claim 1, comprising N-[N-(3,3-dimethylbutyl)-L-$\alpha$-aspartyl]-L-phenylalanine 1-methyl ester and Aspartame, wherein the amount of the N-[N-(3,3-dimethylbutyl)-L-$\alpha$-aspartyl]-L-phenylalanine 1-methyl ester is between 0.1% by weight and 35% by weight based on the total amount of the N-[N-(3,3-dimethylbutyl)-L-$\alpha$-aspartyl]-L-phenylalanine 1-methyl ester and the Aspartame.

3.  A sweetener composition according to claim 1, comprising N-[N-(3,3-dimethylbutyl)-L-$\alpha$-aspartyl]-L-phenylalanine 1-methyl ester, Aspartame and Acesulfame K, wherein the amount of the Acesulfame K is between 0.5% by weight and 85% by weight based on the total amount of the three sweeteners.

4.  A beverage containing the sweetener composition of any one of claims 1 to 3.

5. The beverage of claim 4, which is a carbonated beverage.


**Patentansprüche**

1. Süßmittelzusammensetzung, die N-[N-(3,3-Dimethylbutyl)-L-$\alpha$-aspartyl]-L-phenylalanin-l-methylester (DMB-APM) und Aspartam (APM) und gegebenenfalls Acesulfame K (AceK) in einem Anteil enthält, der eine Süßmittelqualität verleiht, die derjenigen von Saccharose näherkommt als eine der Verbindungen N-[N-(3,3-Dimethylbutyl)-L-$\alpha$-aspartyl]-L-phenylalanin-1-methyl ester, Aspartam und Acesulfame K.

2. Süßmittelzusammensetzung nach Anspruch 1, welche N-[N-(3,3-Dimethylbutyl)-L-$\alpha$-aspartyl]-L-phenylalanin-1-methylester und Aspartam enthält, wobei die Menge des N-[N-(3,3-Dimethylbutyl)-L-$\alpha$-aspartyl]-L-phenylalanin-1-methylesters zwischen 0,1 Gew.-% und 35 Gew.-%, bezogen auf die Gesamtmenge des N-[N-(3,3-Dimethylbutyl)-L-$\alpha$-aspartyl]-L-phenylalanin-1-methylesters und des Aspartams, ist.

3. Süßmittelzusammensetzung nach Anspruch 1, die N-[N-(3,3-Dimethylbutyl)-L-$\alpha$-aspartyl]-L-phenylalanin-1-methylester, Aspartam und Acesulfame K enthält, wobei die Menge von Acesulfame K zwischen 0,5 Gew.-% und 85 Gew.-%, bezogen auf die Gesamtmenge der drei Süßmittel, ist.

4. Getränk, das die Süßmittelzusammensetzung nach einem der Ansprüche 1 bis 3 enthält.

5. Getränk nach Anspruch 4, welches ein kohlensäurehaltiges Getränk ist.


**Revendications**

1. Composition d'édulcorant contenant de l'ester 1-méthylique de N-[N-(3,3-diméthylbutyl)-L-$\alpha$-aspartyl]-L-phénylalanine (DMB-APM) et de l'aspartame (APM) et contenant facultativement de l'acésulfame K (AceK), avec une proportion qui donne à celle-ci une qualité de goût sucré plus proche de celle du sucrose que l'un quelconque de l'ester 1-méthylique de N-[N-(3,3-diméthylbutyl)-L-$\alpha$-aspartyl]-L-phénylalanine, de l'aspartame et de l'acésulfame K.

2. Composition d'édulcorant selon la revendication 1, comprenant de l'ester 1-méthylique de N-[N-(3,3-diméthylbutyl)-L-$\alpha$-aspartyl]-L-phénylalanine et de l'aspartame, dans laquelle la quantité de l'ester 1-méthylique de N-[N-(3,3-diméthylbutyl)-L-$\alpha$-aspartyl]-L-phénylalanine est comprise entre 0,1 % en poids et 35 % en poids sur la base de la quantité totale de l'ester 1-méthylique de N-[N-(3,3-diméthylbutyl)-L-$\alpha$-aspartyl]-L-phénylalanine et de l'aspartame.

3. Composition d'édulcorant selon la revendication 1, comprenant de l'ester 1-méthylique de N-[N-(3,3-diméthylbutyl)-L-$\alpha$-aspartyl]-L-phénylalanine, de l'aspartame et de l'acésulfame K, dans laquelle la quantité de l'acésulfame K est comprise entre 0,5 % en poids et 85 % en poids sur la base de la quantité totale des trois édulcorants.

4. Boisson contenant la composition d'édulcorant de l'une quelconque des revendications 1 à 3.

5. Boisson de la revendication 4, laquelle est une boisson gazeuse.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 8503206 A
- FR 9301103 W
- JP 310311972 B
- JP 56148255 A
- JP 58141760 A
- JP 58220668 A
- JP 57152862 A
- US 4158068 A
- WO 9411391 A